# FASCICULE DE BREVET EUROPEEN

(11) **EP 0 543 709 B1**
(45) Date de publication et mention de la délivrance du brevet: **30.07.1997**
(21) Numéro de dépôt: 92403068.7
(22) Date de dépôt: 13.11.1992
(51) Int. Cl.: A61B 17/86

(54) **Capuchon de protection d'une broche d'ostéosynthèse avec organe pour sa fixation**
Schutzkappe für Knochennagel mit Werkzeug zu deren Befestigung
Protective cap for osteosynthesis pin with instrument for attaching the same

(30) Priorité: 18.11.1991 FR 9114184
(43) Date de publication de la demande: 26.05.1993
(73) Titulaire: OBRY, Christophe, 80680 St. Fucien (FR); SOMEPIC TECHNOLOGIES, 80302 Albert Cedex (FR); SUEUR, Patrick, F-80000 Amiens (FR); LETOT, Patrick, F-76200 Dieppe (FR)
(72) Inventeur: Obry, Christophe, 80680 St. Fucien (FR); Hiot, Jean-Claude, 80300 Bouzincourt (FR); Sueur, Patrick, 80000 Amiens (FR); Letot, Patrick, 76200 Dieppe (FR)
(74) Mandataire: Martin, Jean-Paul

(56) Documents cités:
- EP-A- 323 429
- WO-A-91/16860
- FR-A- 2 633 822
- US-A- 2 081 293
- US-A- 4 976 712

## Description

La présente invention a pour objet un capuchon de protection d'une broche d'ostéosynthèse ainsi qu'un ensemble comportant ce capuchon et un organe de fixation du capuchon sur la broche.

On sait que les broches utilisées en pathologie ostéo-articulaire, notamment pour maintenir alignées deux parties d'un os fracturé, sont introduites à cette fin dans le tissu osseux de part et d'autre de la fracture. Celles-ci ne doivent pas migrer durant la réduction de la fracture. En effet La saillie hors du tissu osseux de la broche non protégée peut provoquer des ruptures tendineuses, des lésions d'éléments vasculo-nerveux, des lésions du revêtement cutané, vecteurs d'infection secondaire, et en outre rendre difficile l'extraction ultérieure de cette broche.

Jusqu'à présent, ou bien la broche est laissée telle quelle en place, avec les risques de complications ultérieures ou de migration, ou bien son extrémité est coiffée d'une olive sertie, ou encore cette extrémité est recourbée ; toutefois cette dernière solution ne donne pas en fait entière satisfaction, car l'on peut mettre en péril l'ostéosynthèse par démontage de la réduction de la fracture.

Le brevet FR 2 633 822 décrit un ensemble comportant un capuchon et un organe de fixation du capuchon sur une broche, le capuchon présentant un logement adapté pour recevoir une extrémité de la broche et un trou taraudé débouchant transversalement dans ce logement, adapté pour recevoir une vis de fixation permettant de solidariser la broche et le capuchon.

Le document EP-A-0 323 429 divulgue une vis de blocage d'une plaque d'ostéosynthèse pour le traitement des fractures. Cette vis comprend une partie filetée, une tête et une tige de préhension solidarisée avec la tête par l'intermédiaire d'une zone de fragilisation formée par un étranglement rendant la tige dissociable de la tête sous l'effet d'un couple de torsion prédéterminé, en fin de vissage.

L'invention a donc pour but de proposer une solution satisfaisante au problème ci-dessus.

Suivant l'invention, l'ensemble formé par le capuchon et l'organe de fixation de celui-ci sur la broche est réalisé conformément à la partie caractérisante de la revendication 1.

Un tel capuchon forme à l'extrémité de la broche une proéminence qui s'oppose efficacement à tout déplacement indésirable de cette broche.

La vis de blocage du capuchon sur la broche fait initialement partie intégrante de son outil de pose, dont elle ne se désolidarise qu'en fin de vissage, par rupture de la zone fragilisée. Cet agencement évite l'utilisation d'un tournevis, laquelle serait particulièrement difficile dans les conditions de l'intervention chirurgicale et compte tenu des faibles dimensions de la vis et du capuchon.

D'autres particularités et avantages de l'invention apparaîtront au cours de la description qui va suivre, faite en référence aux dessins annexés qui en illustrent plusieurs formes de réalisation à titre d'exemples non limitatifs
La figure 1 est une vue mi-élévation mi-coupe longitudinale à échelle agrandie d'une première forme de réalisation de l'ensemble visé par l'invention, comportant un capuchon et un organe de fixation du capuchon sur la broche,
La figure 2 est une vue mi-coupe mi-élévation d'une forme de réalisation du capuchon à échelle agrandie, ainsi que de sa vis de fixation sur la broche et de la partie terminale de l'organe support de la vis.
La figure 3 est une vue en élévation du capuchon suivant la direction de la flèche F de la Fig.2
La figure 4 est une vue en coupe transversale suivant 4/4 de la Fig.5 d'une seconde forme de réalisation du capuchon implant selon l'invention.
La figure 5 est une vue en coupe transversale suivant 5/5 de la Fig.5.
La figure 6 est une vue en élévation d'une première application du capuchon protecteur selon l'invention à la réduction d'une fracture de rotule.
La figure 7 est une vue en élévation postérieure de l'extrémité supérieure d'un cubitus fracturé (olécrane) équipé d'un système de broche et de capuchon implant selon l'invention.
La figure 8 est une vue en élévation schématique d'une application de l'invention à la réduction d'une fracture de l'extrémité inférieure du radius.
La figure 9 est une vue en élévation schématique d'une application de l'invention à une fracture de l'extrémité inférieure du radius.
La figure 10 est une vue en élévation schématique d'une application de l'invention à une fracture malléolaire de cheville.
La figure 11 est une vue en élévation d'une application de l'invention à la fracture d'un métacarpien, ou d'une phalange avec vissage en compression.
La figure 12 est une vue en élévation schématique d'une application de l'invention au traitement chirurgical d'une fracture de scaphoïde ou d'une pseudarthrose du scaphoïde.
La figure 13 est une vue en élévation schématique d'une application de l'invention à l'arthrodèse d'une articulation digitale.
La figure 14 est une vue en élévation schématique d'une application de l'invention à l'arthrodèse d'une articulation métacarpophalangienne du pouce.
La figure 15 est une vue en élévation schématique d'une application de l'invention au blocage temporaire d'une articulation digitale.
La figure 16 est une vue en élévation schématique d'une application de l'invention dans laquelle une broche munie de son capuchon protecteur est utilisée comme tutrice d'un orteil en griffe après correction chirurgicale de la déformation.
La figure 17 est une vue analogue à la Fig.16 dans laquelle la broche est utilisée comme tutrice d'un orteil en marteau.

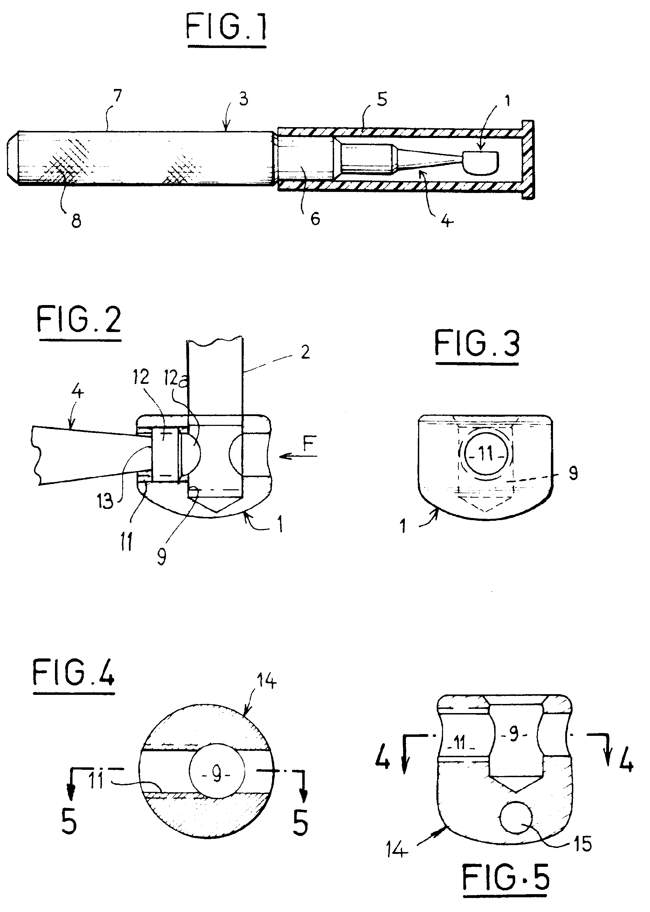

Le dispositif représenté à la Fig.1 comporte un capuchon 1 de protection d'une broche d'ostéosynthèse telle que 2 (Fig.2), un organe 3 de fixation du capuchon 1 sur la broche, cet organe étant pourvu d'une partie terminale 4 de support du capuchon 1, et enfin un capot 5 de protection coiffant la partie terminale 4 et le capuchon 1. Le capot 5, réalisé par exemple en toute matière plastique appropriée, présente une forme tubulaire et peut être emmanché à friction sur une base cylindrique 6 coaxiale à la partie terminale 4. Cette base 6 est prolongée par un manche 7 de manutention manuelle, dont la surface est de préférence revêtue d'aspérités, par exemple un moletage 8.

Le capuchon 1, représenté en détail aux Fig.2 et 3, comporte un logement 9 formé par un trou borgne adapté pour recevoir une extrémité de la broche 2. Dans le capuchon implant 1, est de plus ménagé un trou taraudé 11 débouchant transversalement dans le logement 9, et adapté pour recevoir une vis 12 permettant de solidariser la broche 2 et le capuchon 1. Dans le mode de réalisation représenté, le trou 11 traverse de part en part le capuchon 1, perpendiculairement à l'axe du logement cylindrique 9, seule sa partie destinée à recevoir la vis 12 étant taraudée.

La vis 12 présente une tête 12a adaptée pour épouser la surface cylindrique de la broche 2. Elle est réalisée monopièce avec la partie terminale 4 du manche-vis 3, et présente, à sa jonction avec l'extrémité de cette partie 4, une zone fragile 13 permettant sa rupture en fin de vissage et ainsi la désolidarisation de la vis 12 par rapport au col formant la partie terminale 4.

La mise en place du capuchon 1 et sa fixation à la broche 2 s'effectuent très simplement, de la manière suivante.

Après avoir enlevé le capuchon 5, le chirurgien, tenant en main le manche 7, approche le capuchon 1 de l'extrémité de la broche 2 qu'il introduit dans le logement 9, jusqu'à ce que sa pointe vienne en butée sur le fond de ce logement. Puis, la vis 12 n'étant que partiellement enfoncée dans son trou 11, le chirurgien commence à la visser en faisant tourner l'outil 3 comme un tournevis. En fin de vissage, la tête 12a de la vis 12 vient s'appliquer étroitement contre la broche 2 qu'elle bloque en translation et en rotation par rapport au capuchon 1. Puis, en forçant sur le manche 7, le chirurgien provoque la rupture de la zone fragile 13, située à ce moment à l'intérieur du trou taraudé 11. La vis 12 est alors en place et fixe le capuchon 1 à la broche 2.

La seconde forme de réalisation du capuchon 14, représentée aux Fig.4 et 5, diffère de la précédente par le fait que le corps du capuchon est plus volumineux et perforé par un alésage 15, orienté de préférence perpendiculairement à l'axe du logement 9 et donc à la broche 2. L'alésage 15 traverse de part en part le capuchon 14, et permet le passage d'un fil de cerclage ou d'un haubanage osseux, comme cela sera expliqué plus en détail ci-après.

Bien entendu, pour une dimension déterminée du capuchon 1 ou 14, la cavité 9 recevant la broche 2 peut avoir un diamètre variable, adapté à celui de la broche, laquelle peut de plus être non filetée, ou bien partiellement ou totalement filetée.

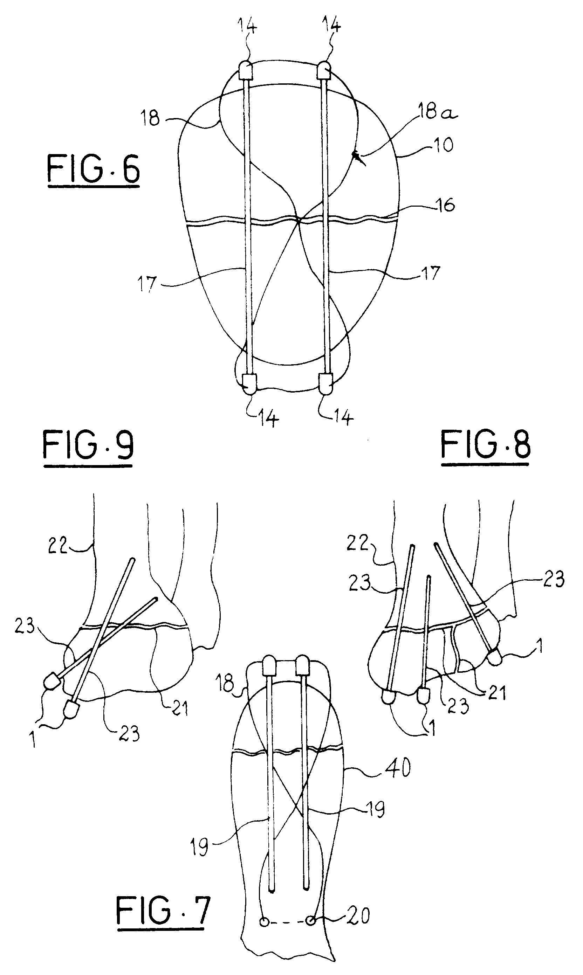

On décrira maintenant en référence aux Fig.6 à 17, un certain nombre d'exemples de mise en oeuvre de l'invention.

La Fig.6 illustre l'application de l'invention à la fracture 16 d'une rotule 10 : le dispositif comprend deux broches 17 dont chacune des deux extrémités est coiffée d'un capuchon implant 14 dans les trous 15 desquels passe un fil 18, par exemple en acier. Ce fil, dont les deux bouts sont noués en 18a, forme un cerclage ou haubanage de maintien en compression des deux parties de la rotule fracturée.

La Fig.7 est une vue postérieure de l'extrémité supérieure d'un cubitus fracturé 40 (olécrane), équipée de deux broches 19 munies chacune d'un capuchon 14, traversé par un fil d'acier 18 dont les extrémités ont été nouées de façon à former une boucle fermée qui traverse un canal osseux 20.

La Fig.8 montre l'extrémité inférieure d'un radius 22, à deux lignes 21 de fracture, équipé d'un dispositif conforme à l'invention à trois broches 23, dont les extrémités saillantes du radius 22 sont coiffées chacune par un capuchon 1. L'agencement représenté à la Fig.8 constitue un embrochage intrafocal selon Kapandji

La Fig.9 montre un radius 22 dont l'extrémité inférieure présente une ligne unique de fracture 21, et qui est munie de deux broches 23 croisées, dont les extrémités saillant à l'extérieur du radius sont coiffées chacune d'un capuchon 1. Il s'agit ici d'un embrochage styloïdien.

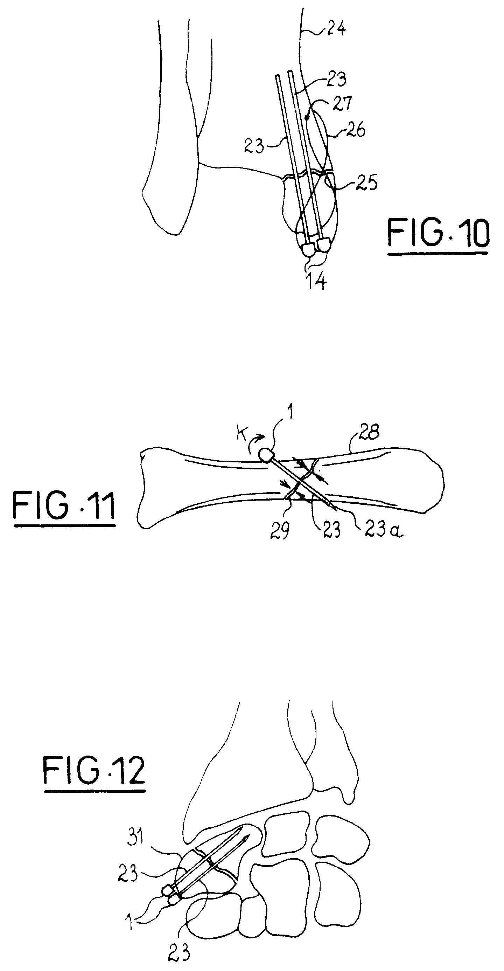

La Fig.10 illustre un montage conforme à l'invention de deux broches 23 sur une cheville 24 ayant subi une fracture malléolaire interne 25. Chaque broche 23 a son extrémité saillant extérieurement à la cheville 24 coiffée par un capuchon 14 traversé par un fil 26, par exemple en acier, formant une boucle fermée qui traverse un canal transosseux tibial 27. Un tel dispositif est applicable à la malléole interne ou externe.

La Fig.11 montre un métacarpien 28 dont la fracture 29 est traversée par une broche 23. L'extrémité distale 23a de la broche 23 est filetée et traverse la corticale, tandis que l'extrémité proximale est revêtue d'un capuchon protecteur 1. L'ensemble de la broche 23 et du capuchon 1 est vissé, au moyen d'une pince comme indiqué par la flèche K, pour développer une compression au niveau du foyer de fracture. Cette compression est induite par le léger déplacement en translation de la broche entraîné par sa rotation. Ceci est applicable aux autres os de l'appareil locomoteur.

La Fig.12 montre un scaphoïde carpien 31 fracturé, muni de deux broches 23 dont les extrémités extérieures au scaphoïde sont coiffées d'un capuchon 1. Un tel dispositif est applicable au traitement chirurgical des pseudarthoses du scaphoïde carpien, en y associant ou non une greffe osseuse, aux autres osselets du carpe ou du tarse, au tarse dans les fractures-luxations intéressant le tarse et le métatarse, ou à toute autre pathologie fracturaire du tarse et du métatarse

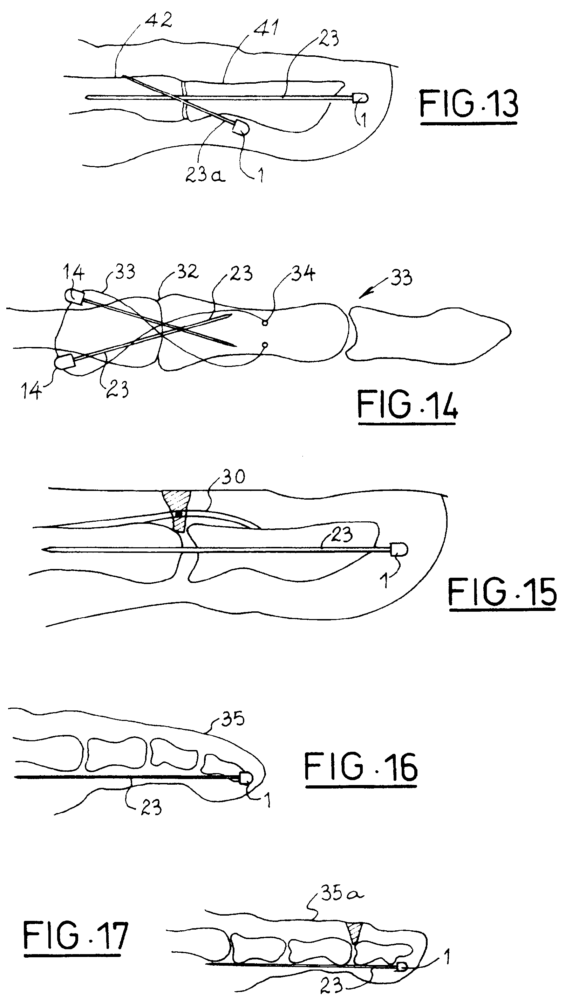

La Fig.13 est un exemple d'application de l'invention à une arthrodèse (soudure l'une à l'autre des deux parties de l'articulation) d'articulation digitale, telle que l'interphalangienne distale (ou proximale). Les deux parties 41, 42 de l'os fracturé sont maintenues en alignement par une broche axiale 23 (lisse ou filetée) complétée par une petite broche 23a antirotation, oblique par rapport à la broche 23 de façon à empécher l'une des deux parties de l'os de tourner par rapport à l'autre. Chacune des deux broches est coiffée d'un capuchon 1.

La Fig.14 illustre une arthrodèse de l'articulation métacarpophalangienne 32 du pouce 33, ou de l'articulation métatarso-phalangienne du gros orteil. Le dispositif comprend ici deux broches 23 munies chacune d'un capuchon 14 traversé par un fil 33 empruntant un passage transosseux 34. Un tel dispositif est également applicable à l'articulation interphalangienne du pouce et du gros orteil.

La Fig.15 montre une protection d'une suture de l'appareil extenseur distal (30) avec blocage transitoire de l'articulation interphalangienne distale par une broche 23 munie d'un capuchon 1 (cette broche est à maintenir 4 à 6 semaines).

La Fig.16 montre un orteil 35 en griffe, dans le canal digital duquel a été introduite, au ras de l'os, une broche tutrice 23 munie d'un capuchon 1, après une arthroplastie modelante de l'articulation interphalangienne proximale de l'orteil.

La Fig.17 montre un orteil en marteau 35a sur lequel a été effectuée une téno-dermodèse interphalangienne distale, puis dans le canal digital duquel on a mis en place temporairement une broche tutrice 23.

En plus des avantages déjà mentionnés, le capuchon protecteur et l'ensemble dont il fait partie présentent les avantages suivants.
1 - Le capuchon 1, 14 protège les tissus avoisinants, en s'opposant,à tout déplacement intempestif de la broche ou migration.
2 - Le capuchon protecteur, mis en place sous le revêtement cutané, est aisément repérable manuellement par palpation, ce qui facilite l'ablation de la broche par le chirurgien. Ainsi est supprimée pour ce dernier la contrainte de devoir utiliser un amplificateur de brillance, ce qui lui procure un gain de temps et lui évite les radiations émises par l'amplificateur de brillance.
   Corrélalivement, l'extraction des broches après consolidation de la fracture n'entraîne pas de lésion importante par la recherche intempestive des broches, et rend donc moins "invasive" l'intervention chirurgicale que jusqu'à présent. L'ablation des broches munies d'un capuchon protecteur selon l'invention est ainsi rendue relativement aisée "à distance" de la mise en place (c'est-à-dire au bout d'un certain temps).
3 - L'implant constitué par le capuchon protecteur selon l'invention permet au chirurgien de choisir une broche de la longueur souhaitée. Il suffit en effet de mettre en place la broche, de la couper à la longueur choisie et d'y fixer le capuchon protecteur. Ceci présente un avantage considérable par rapport aux broches auto-cassables à tailles prédéterminées de la technique antérieure. Ces broches, protégées par un embout, sont le plus souvent disponibles de 0,5cm en 0,5cm. Elles exposent également à l'inconvénient d'une rupture en aval de l'olive protectrice, faisant ainsi perdre tous les avantages d'un embout protecteur de la broche. En outre, la prédétermination des longueurs peut obliger à faire dépasser le bout pointu de la broche à travers la corticale opposée, ce qui est une source d'irritation des tissus avoisinants.
4 - L'implant protecteur selon l'invention peut permettre de créer, au sein d'un foyer de fracture ou d'arthrodèse, un certain degré de compression, par l'utilisation de broches filetées en leurs extrémités distales, le capuchon étant placé en position proximale (Fig.11).
5 - Le capuchon protecteur 1, 14 selon l'invention est d'une grande simplicité d'utilisation et peut s'adapter à des broches totalement, partiellement ou non filetées.

L'utilisation de fils métalliques ou textiles constituant des haubanages est intéressante dans un certain nombre d'applications : fracture de rotule (Fig.6), fracture de l'olécrane (Fig.7), les extrémités proximales des broches protégées par les capuchons 14 permettant la mise en place d'un hauban 18. Ces haubanages sont également utilisables dans d'autres applications telles que celles déjà mentionnées de manière non limitative (Fig.10 et 14).

## Revendications

1. Ensemble comportant un capuchon (1, 14) de protection d'une broche (2...) d'ostéosynthèse, et un organe (3) de fixation du capuchon sur la broche, par l'intermédiaire d'une partie terminale (4) dudit organe de fixation, le capuchon comportant un logement (9) adapté pour recevoir une extrémité de la broche, et un trou taraudé (11) débouchant transversalement dans ce logement, adapté pour recevoir une vis (12) de fixation prolongeant la partie terminale (4) et permettant de solidariser la broche et le capuchon, caractérisé en ce que la vis (12) présente, à sa jonction avec l'extrémité de ladite partie terminale, une zone fragile (13) permettant sa rupture en fin de vissage et son blocage dans le trou transversal (11).

2. Ensemble selon la revendication 1, caractérisé en ce que le capuchon (14) est perforé par un alésage (15) permettant le passage d'un fil (18, 33...) de cerclage d'un os (10, 40...) destiné à recevoir la broche (2) équipée de ce capuchon (1, 14).

3. Ensemble comportant un capuchon (1, 14) selon l'une des revendications 1 et 2 et un organe (3) de fixation du capuchon sur la broche (2), pourvu d'une partie terminale (4) réalisée monopièce avec la vis (12), une zone fragilisée (13) de rupture en fin de vissage étant ménagée entre la vis et ladite partie terminale.

4. Ensemble selon la revendication 3, caractérisé en ce que la zone fragilisée (13) est un rétrécissement de la partie terminale (4), formant un col à la jonction avec la vis (12).

5. Ensemble selon la revendication 4, caractérisé en ce que l'organe de fixation (3) est un manche, de préférence revêtu d'aspérités (8), par exemple un moletage.

6. Ensemble selon la revendication 5, caractérisé en ce qu'il comprend un capot protecteur (5) coiffant la partie terminale (4) et le capuchon (1).

## Claims

1. Arrangement comprising a protective cap (1, 14) for an osteosynthesis pin (2...) and an instrument (3) for attaching the cap to the pin, through the intermediary of a terminal part (4) of said fixing instrument, the cap comprising a housing (9) adapted to receive one end of the pin, and a tapped hole (11) opening out transversely in this housing, adapted to receive a fixing screw (12) extending the terminal part (4) and making it possible to interconnect the pin and the cap, characterised in that the screw (12) comprises, at its junction with the end of said terminal part, a fragile area (13) allowing its rupture at the end of screwing and its locking in the transverse hole (11).

2. Arrangement according to Claim 1, characterised in that the cap (14) is perforated by a bore (15) allowing the passage of a wire (18, 33) for circling a bone (10, 40) intended to receive the pin (2) equipped with this cap (1, 14).

3. Arrangement comprising a cap (1, 14) according to one of Claims 1 and 2 and an instrument (3) for attaching the cap to the pin (2), provided with a terminal part (4) made in one piece with the screw (12), a fragile area (13) for rupture at the end of screwing being provided between the screw and said terminal part.

4. Arrangement according to Claim 3, characterised in that the fragile area (13) is a narrow portion of the terminal part (4), forming a neck at the junction with the screw (12).

5. Arrangement according to Claim 4, characterised in that the attachment instrument (3) is a handle preferably covered with roughness (8), for example knurling.

6. Arrangement according to Claim 5, characterised in that it comprises a protective cover (5) covering the terminal part (4) and the cap (1).

## Patentansprüche

1. Gesamtheit aus einer Schutzkappe (1, 14) für einen Knochennagel (2 ...) und einem Element (3) zum Befestigen der Kappe auf dem Nagel mittels eines Endabschnittes (4) des Befestigungselementes, wobei die Kappe eine Ausnehmung (9), die dazu ausgelegt ist, ein Ende des Nagels aufzunehmen, und ein mit einem Gewinde versehenes Loch (11), das quer in diese Ausnehmung mündet, aufweist, welches dazu ausgelegt ist, eine Befestigungsschraube (12) aufzunehmen, die den Endabschnitt (4) verlängert und die es ermöglicht den Nagel und die Kappe fest miteinander zu verbinden, dadurch gekennzeichnet, daß die Schraube (12) an ihrer Verbindung mit dem Ende des Endabschnittes eine Bruchzone (13) aufweist, die ihr Abreißen am Schluß des Einschraubens und ihr Sperren in dem Querloch (11) ermöglicht.

2. Gesamtheit nach Anspruch 1, dadurch gekennzeichnet, daß die Kappe (14) von einer Bohrung (15) durchsetzt ist, welche den Durchgang eines Fadens (18, 33) zum Einbinden eines Knochens (10, 40 ...) ermöglicht, der den mit der Kappe (1, 14) versehenen Nagel (2) erhalten soll.

3. Gesamtheit aus einer Kappe (1, 14) nach einem der Ansprüche 1 und 2 und einem Element (3) zum Befestigen der Kappe auf dem Nagel (2), das mit einem Endabschnitt (4) versehen ist, der einstückig mit der Schraube (12) ausgebildet ist, wobei eine Bruchzone (13) für das Abreißen am Schluß des Einschraubens zwischen der Schraube und dem Endabschnitt angeordnet ist.

4. Gesamtheit nach Anspruch 3, dadurch gekennzeichnet, daß die Bruchzone (13) eine Verengung des Endabschnittes (4) ist, die einen Hals an der Verbindungsstelle mit der Schraube (12) bildet.

5. Gesamtheit nach Anspruch 4, dadurch gekennzeichnet, daß das Befestigungselement (3) eine Haltestange ist, bevorzugt mit rauhen Stellen (8) überzogen, beispielsweise einer Rändelung.

6. Gesamtheit nach Anspruch 5, dadurch gekennzeichnet, daß sie eine Schutzhaube (5) aufweist, welche den Endabschnitt (4) und die Kappe (1) abdeckt.
